(19) Europäisches Patentamt European Patent Office Office européen des brevets

(11) **EP 3 125 950 B1**

(12) **EUROPEAN PATENT SPECIFICATION**

(45) Date of publication and mention of the grant of the patent:
**29.04.2026 Bulletin 2026/18**

(21) Application number: **15712930.5**

(22) Date of filing: **31.03.2015**

(51) International Patent Classification (IPC):
**A61K 49/22** $^{(2006.01)}$

(52) Cooperative Patent Classification (CPC):
**A61K 49/223**

(86) International application number:
**PCT/EP2015/056954**

(87) International publication number:
**WO 2015/150354 (08.10.2015 Gazette 2015/40)**

(54) **ULTRASOUND PRECURSOR PREPARATION METHOD**

VERFAHREN ZUR HERSTELLUNG VON EINEM PRECURSOR EINES ULTRASCHALLKONSTRASTMITTELS

PROCÉDÉ POUR PRÉPARER UN PRÉCURSEUR D'UN AGENT DE CONTRASTE POUR L'ÉCHOGRAPHIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2014 GB 201405735**

(43) Date of publication of application:
**08.02.2017 Bulletin 2017/06**

(73) Proprietor: **GE Healthcare AS**
**0401 Nydalen (NO)**

(72) Inventors:
• **HENRIKSEN, Ingrid**
**N-0401 Oslo (NO)**
• **KVALE, Svein**
**N-0401 Oslo (NO)**
• **TOKERUD, Ole, Johannes**
**N-0401 Oslo (NO)**
• **PER, Sontum**
**N-0401 Oslo (NO)**
• **SWAERD-NORDMO KISERUD, Marit**
**N-0401 Oslo (NO)**

(74) Representative: **Coles, Andrea Birgit et al**
**Kilburn & Strode LLP**
**Lacon London**
**84 Theobalds Road**
**London WC1X 8NL (GB)**

(56) References cited:
**WO-A1-99/08715    WO-A2-99/08716**

## Description

Field of the Invention.

The present invention relates to a method of preparation of a commercial scale batch of vials of an ultrasound contrast agent precursor comprising phospholipid-stabilised perfluorobutane microbubbles. The method is amenable to commercial scale manufacture, and provides more consistent vial-to-vial yields and size distribution of said microbubbles. Also provided are methods of preparing kits and ultrasound contrast agents using the method of the invention.

Background to the Invention.

Ultrasound contrast agents based on phospholipid-stabilised microbubbles of perfluorocarbons (e.g. perfluoropropane or perfluorobutane), are well known in the art [see e.g. Wheatley et al, J.Drug Del.Sci.Technol., 23(1), 57-72 (2013)].

WO 97/29782 teaches that ultrasound contrast agent precursors which are stable at room temperature can be prepared by lyophilization of perfluorocarbon microbubbles in the presence of a freeze-drying stabiliser chosen from: sucrose, maltose, trehalose, raffinose or stachyose, preferably sucrose.

WO 99/03558 discloses a method of selecting ultrasound contrast gas microbubbles form a polydisperse mixture, to give such microbubbles having a tighter size distribution (i.e. monodisperse). The method described involves flotation and re-suspension.

WO 99/08715 discloses a process for the preparation of a pharmaceutical composition comprising an aqueous dispersion of gas-containing vesicles the membranes whereof comprise an amphiphilic membrane-forming material, said process comprising:

> (i) generating a liquid dispersion of gas-containing vesicles from a mixture comprising an amphiphilic membrane forming material;
> (ii) lyophilizing a liquid dispersion of said gas-containing vesicles;
> (iii) reconstituting the lyophilized product of step (ii) with a sterile aqueous liquid to produce an aqueous dispersion of gas-containing vesicles; and
> (iv) treating the aqueous dispersion product of step (i) or step (iii) or the lyophilized product of step (ii) to produce a substantially aggregate-free sterile aqueous dispersion of gas-containing vesicles.

WO 99/08715 teaches that the time between steps (i) and (ii) should be minimised in order to suppress the agglomeration of the microbubbles to form unwanted larger species. WO 99/08715 does not, however, teach that the gas-containing vesicles should be subjected to size control before lyophilisation, that a cryoprotectant/lyoprotectant must be used before the lyophilisation step, or dispensing into multiple individual vials in a batch manufacture process.

WO 99/08716 relates to the preparation of gas-containing contrast agents, such as a fluorocarbon-containing lyophilised contrast agent.

EP 1228770 A1 teaches a process for the preparation of lyophilised ultrasound contrast agents comprising gas microbubbles, in which the vials of lyophilised precursor are sealed under a reduced pressure of the headspace gas. The reduced pressure is said to assist in controlling the particle size in the aqueous microbubble composition formed post-reconstitution of the precursor vial.

WO 2004/069284 addresses the problem of obtaining phospholipid-stabilised gas microbubble ultrasound contrast agents of a defined size distribution. WO 2004/069284 teaches that a substantially water-immiscible organic solvent should be used in the microbubble preparation emulsion, prior to lyophilisation. WO 2004/069284 teaches that the microbubbles obtained upon reconstitution of the lyophilised precursor have a relatively small diameter, and a narrow size distribution.

Solis et al [Int.J.Pharmaceut., 396(1-2), 30-38 (2010)] studied the effect of excipients, including lyoprotectants, on lyophilised perfluoropropane microbubble precursors. They concluded that glucose was the best lyoprotectant, closely followed by trehalose, with both being superior to sucrose or mannitol.

Feshitan et al [J. Coll.Interf. Sci., 329, 316-324 (2009)] report that, in contrast to surfactant-coated microbubbles, lipid-coated microbubbles are stable after centrifugation, and that the lipid shell is highly viscous and relatively impermeable to gases. Feshitan *et al* report that perfluorobutane microbubbles in the 4-5 $\mu$m size range were stable for at least 2-days, but tended to disintegrate into smaller size microbubbles (1-2 $\mu$m) after 2-weeks.

There is still a need for ultrasound lyophilised precursor preparation methods which give control over the vial-to-vial variation of the microbubble concentration and size distribution, yield and other important characteristics of the contrast agent. Such methods need to be amenable to commercial scale manufacture, and deliver precursor products with a uniformity of vial content and safety profile which satisfy the regulatory requirements (e.g. U.S. Pharmacopeia and ICH Guidelines).

The Present Invention.

Conventional microbubble ultrasound contrast agent preparation methods such as sonication, and high shear emulsification provide good yields and low cost production, with the capability of scale-up to commercial manufacture. They suffer from the disadvantage of difficulty in controlling the size distribution of the microbubbles. Whilst other preparation techniques have been developed which permit the preparation of such microbubbles with a tighter size distribution, these alternative methods are either lower-yielding, involve high cost equipment or are too slow to scale-up to commercial production methodology.

The present invention seeks to provide methods of commercial scale manufacture, which also give adequate control over the vial-to-vial variation of the microbubble concentration, size distribution, yield and other important characteristics of the contrast agent.

The present inventors have found that, in production of batch sizes of thousands of vials of microbubble contrast agents, two key parameters are important to control variability: (a) microbubble size range prior to lyophilisation; and (b) the elapsed time between dispensing into a given vial and when the vial contents are frozen at -40 to -70 °C ("holding time").

The present invention provides a method of controlling both parameters, with consequent advantages of:

(i) optimal yield and concentration of microbubbles in each vial;
(ii) control over uniformity of content from vial to vial.

The contrast agent precursors of the present invention have also been found to be less sensitive towards multivalent cations.

Without wishing to be bound by theory, the present inventors believe that, due to the high density increment between the liquid matrix (e.g. sucrose solution) and the gas-filled microbubbles, the microbubbles tend to segregate rapidly to the top of the liquid volume ("creaming"). It is this creaming that needs to be minimized and controlled in order to provide benefits (i) and (ii) described above. It is believed likely that a high concentration of microbubbles in the surface layer impairs the drying of the cake during lyophilisation, and/or that too high a local concentration depletes the survival rate of microbubbles, as they are trapped partially outside the final sucrose structure i.e. as holes in the surface rather than fully embedded gas pockets.

Detailed Description of the Invention.

In a first aspect, the present invention provides a method of preparation of a commercial scale batch of vials of an ultrasound contrast agent precursor which comprises a lyophilised composition of microbubbles of perfluorobutane stabilised by hydrogenated egg phosphatidylserine in sucrose; wherein said batch size is in the range 500 to 80,000 vials; wherein said process comprises:

(i) provision of an aqueous suspension of said perfluorobutane microbubbles stabilised by hydrogenated egg phosphatidylserine in an aqueous solution;
(ii) adjusting the size of said microbubbles to a median size in the range 2 to 5 $\mu$m;

(iii) diluting the size-adjusted suspension from step (ii) with a sterile aqueous sucrose solution to give a final sucrose concentration of 5-20 % w/v;
(iv) dispensing an aliquot of the composition from step (iii) into a vial to give a filled vial, wherein multiple vials are dispensed in parallel;
(v) within a holding time period of 1.0 to 2.75 minutes for each filled vial after the dispensing of step (iv) is complete, cooling each such filled vial to a temperature of -40 °C to -70 °C, wherein multiple filled vials ae cooled in parallel wherein cooling is achieved by loading into a freeze-drier apparatus, in which the shelves are pre-cooled to -60 °C;
(vi) repeating steps (iv) and (v) as necessary, until the desired number of cooled, filled vials has been obtained;
(vii) freeze-drying the cooled, filled vials from steps (v) and (vi);
(viii) back-filling the headspace gas in the freeze-dried vials from step (vii) with perfluorobutane;
(ix) sealing the vials from step (viii) with a closure.

The term "contrast agent" has its conventional meaning in the field of *in vivo* medical imaging, and refers to an agent in a form suitable for mammalian administration, which assists in providing clearer images in the region or organ of interest than could be obtained by imaging the mammalian subject alone. By the term "subject" is meant a mammal *in vivo*, preferably the intact mammalian body *in vivo*, and more preferably a living human subject. By the phrase "in a form suitable for mammalian administration" is meant a composition which is sterile, pyrogen-free, lacks compounds which produce toxic or adverse effects, and is formulated at a biocompatible pH (approximately pH 4.0 to 10.5). Such compositions lack particulates which could risk causing emboli *in vivo*, and are formulated so that precipitation does not occur on contact with biological fluids (e.g. blood). Such compositions also contain only biologically compatible excipients, and are preferably isotonic.

As with other *in vivo* imaging agents, the contrast agent is designed to have minimal pharmacological effect on the mammalian subject to be imaged. Preferably, the contrast agent can be administered to the mammalian body in a minimally invasive manner, i.e. without a substantial health risk to the mammalian subject when carried out under professional medical expertise. Such minimally invasive administration is preferably intravenous administration into a peripheral vein of said subject, without the need for local or general anaesthetic.

The term "microbubble" has its conventional meaning in the field of *in vivo* ultrasound imaging, and refers to a gas microbubble of diameter typically between 0.5 and 10 $\mu$m. At sizes above 7 $\mu$m there is a substantial risk of retention (embolization) in the lung capillaries. Such microbubbles are similar in size to a red blood cell, which allow them to display similar characteristics in the microvessels and capillaries throughout the mammalian body [Sirsi et al, Bubble Sci.Eng.Technol., 1(1-2), 3-17 (2009)].

Suitable microbubbles of the present invention are stabilised by a phospholipid shell or coating - as described by Sirsi *et al* (above), in particular the phospholipids present in hydrogenated egg phosphatidylserine (H-EPS). The phospholipids present in H-EPS are primarily phosphatidylserine and phosphatidic acid [Hvattum et al, J.Pharm.Biomed. Anal, 42, 506-512 (2006)].

Perfluorobutane ("PFB") has its standard chemical meaning, and is also referred to in the context of medical uses as perflubutane. The chemical formula of perfluoro-n-butane is $CF_3CF_2CF_2CF_3$ or $C_4F_{10}$, with a boiling point of -2.2 °C. Commercial perfluoro-n-butane contains a minor amount (typically 2-4%) of the perfluoro-iso-butane isomer, i.e. $CF_3CF(CF_3)CF_3$.

The term "aqueous suspension" refers to a suspension of the microbubble in an aqueous solvent, which comprises water and/or water-miscible solvents. The aqueous solvent is preferably a biocompatible carrier. By the term "biocompatible carrier" is meant a fluid, especially a liquid, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous buffer solution comprising a biocompatible buffering agent (e.g. phosphate buffer); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like). Preferably the biocompatible carrier is pyrogen-free water for injection, isotonic saline or phosphate buffer. Hence the aqueous suspension suitably excludes water-immiscible organic solvents.

The term "precursor" refers to a convenient or kit form of the contrast agent, designed so that, upon reconstitution, it readily forms the desired ultrasound contrast agent.

The term "filled vial" refers to a charged vial, a vial into which has been dispensed an aliquot of the composition, i.e. a dispensed vial. The vial is not physically full, since the vial volume will typically be 10 mL, and the dispensed volume about 2 mL - leaving a headspace gas above the composition. The term 'headspace gas' has its conventional meaning, and refers to the gas within the vial over the lyophilised composition.

The phrase "holding time" refers to the time delay after filling, i.e. the dispensing of an individual vial in step (iv), and before initiating the cooling of the filled vial to -40 to -70 °C.

The microbubble preparation of step (i) of the method of the first aspect can be carried out by a variety of methods known in the art, including:

(i) sonication;
(ii) high shear emulsification;
(iii) membrane emulsification;
(iv) coaxial electrohydrodynamic atomisation (CEHDA); and
(v) microfluidic devices;
(vi) ink jet printing.

These methods are described by Stride et al [Soft Matter, 4, 2350-2359 (2008)] and references therein. Further details of microbubble preparation methods are provided by Unnikrishnan et al [Am.J.Roentgenol., 199(2), 292-299 (2012)].

Ink-jet printing is reported by Stride *et al* (above) to be more suited to liquid-filled particles, so is less suitable for the PFB microbubbles of the present invention. Sonication is less preferred, since it tends to give a wide range of bubble sizes, and microfluidic techniques suffer from problems with slow production rates unsuitable for commercial manufacture [Wang et al, Ultraso.Med.Biol., 39(5), 882-892 (2013)].

A preferred production method of the present invention is high shear emulsification, preferably using a rotor stator mixer - since that confers a greater degree of size control, is amenable to large batch production, and provides a static size distribution. The microbubbles are formed by agitating a sterile, pyrogen-free lipid suspension in the presence of the perfluorocarbon (here PFB) for several seconds. Example 1 provides a rotor stator microbubble preparation method. Rotor-stator mixers are described in Rodgers et al [Chem.Eng.Res.Rev., 90(3), 323-327 (2012) and in Emulsion Formation and Stability [T.F.Tadros (Ed), Wiley VCH (2013)] - see especially Pacek et al Chapter 5 Emulsification in Rotor-Stator Mixers, p. 127-167.

The microbubble size adjustment of step (ii) of the method of the first aspect can be carried out by a variety of methods known in the art, including:

(a) one or more cycles of flotation and re-suspension; or
(b) differential centrifugation; or
(c) cross flow filtration with large pores; or
(d) field-flow fractionation.

Flotation and re-suspension is described by Kvale et al [Separat.Technol., 6, 219-226 (1996)], and in present Example 2. Differential centrifugation to separate and isolate sub-populations of lipid-coated perfluorobutane microbubbles in the size ranges 1-2 $\mu$m and 4-5 $\mu$m is described by Feshitan et al [J.Coll.Interf.Sci., 329, 316-324 (2009)].

Phospholipid-stabilised perfluorobutane microbubbles, such as the agent Sonazoid™ are both flexible and stable. They are thus capable of passing through a 5

μm filter without significant changes in the size distribution [Sontum, Ultraso.Med.Biol., 34(5), 824-833 (2008)]. This, and the fact that such filtration cannot remove microbubbles that are too small, means that passage through a filter is, in itself, not a suitable method for the size adjustment of step (ii) of the present invention.

Certain preparation techniques aim to provide microbubbles of a defined, tighter size distribution. In such cases, steps (i) and (ii) of the method of the first aspect are, in effect, carried out concurrently. Those preparation techniques are CEHDA and microfluidic devices. Hence, in an alternative embodiment, the first aspect includes the option of carrying out steps (i) and (ii) concurrently, as opposed to sequentially.

The phrase "cooling each such filled vial to a temperature of -40 °C to -70 °C" refers to the cooling of the vial and contents. Freezing of the vial contents will occur, but with some degree of variation, since some vials may supercool on the freeze-drier shelves and hence take longer for the vial contents to freeze.

The method of the present invention is for use in commercial scale production, with numbers of vials in the range 500 to 80,000 in a production batch, typically around 35,000 to 40,000. In such large scale, commercial manufacture, consistency from vial-to-vial, i.e. minimising vial-to-vial variation is important. Hence, the aliquots dispensed in step (iv) are preferably of the same volume, and more preferably controlled within a tolerance of ± 3%.

With such large numbers of vials, the present inventors have found that it is important to control the size distribution of the microbubbles, and the hold time before lyophilisation. Rapid freezing in an aqueous sucrose solution as per step (v) has been found to be important to control uniformity of content, especially microbubble concentration, from vial to vial. Such rapid cooling can be carried out by methods known in the art, such as: immersion in a bath of liquid nitrogen; immersion in an organic solvent/dry ice cold bath; or cold shelves or environments within a freeze-drier apparatus.

In such circumstances of commercial batch sizes, it has been found that is not viable to proceed in a stepwise manner and wait until all vials are filled, before all the dispensed/filled vials are then frozen. This contravenes the conventional wisdom that, once isolated, lipid-coated PFB microbubbles are stable for over 2 days [Feshitan et al, J.Coll.Interf.Sci., 329, 316-324 (2009)]. The present inventors have thus found, that in order to control uniformity of the batch, the following key parameters must both be controlled:

    (i) the median microbubble size prior to lyophilisation; and
    (ii) the 'holding time' - which must be minimised to a period of 1.0 to 2.75 minutes.

Further details are given in the supporting Examples and Figures (below).

In step (vi), the "desired number" refers to the total number of vials in the particular production batch of contrast agent precursor. Thus, step (vi) is intended to cover the situation whereby the need to fulfil the holding time limitation of step (v) means that, for a given batch size of vials, many repeat cycles of dispensing and freezing may be required until the entire batch of vials has been filled and cooled, and is ready to be lyophilised as per step (vii).

The terms "comprising" or "comprises" have their conventional meaning throughout this application and imply that the agent or composition must have the essential features or components listed, but that others may be present in addition. The term 'comprising' includes as a preferred subset "consisting essentially of" which means that the composition has the components listed without other features or components being present.

In the back-filling step (viii) the headspace gas is filled to approximately atmospheric pressure, preferably just below atmospheric pressure to facilitate stoppering of the vials, as is known in the art.

Preferred embodiments.

The method of the first aspect provides a concentration of microspheres in the range 14-18 μl/mL, and a size distribution of microbubbles (median size) in the range 2.7-3.5 μm.

In the method of the first aspect, the holding time period of step (v) is controlled such that it falls within a time range of 1.0 to 2.75 minutes for each filled vial.

In the method of the first aspect, the lyophilized precursor composition is preferably sterile.

In the method of the first aspect, the batch size is in the range 500 to 80,000, more preferably 1,000 to 50,000, most preferably 5,000 to 45,000 vials to be filled with contrast agent precursor. A batch size of about 35,000 to 40,000 vials is ideal.

The temperature to which the filled vials are cooled in step (v) is preferably about -60 °C. Rapid cooling is achieved by loading into a freeze-drier apparatus, in which the shelves are pre-cooled to -60 °C.

In the method of the first aspect, the sucrose concentration of step (iii) is preferably 8-12% w/v, more preferably about 10% w/v, most preferably 92 mg/mL. The sucrose acts as a lyoprotectant/cryoprotectant, and has been shown to form a matrix wherein the lipid-stabilised PFB microbubbles are trapped within the lyophilised sucrose of the precursor, such that the microbubble size and concentration are predefined - and not influenced by the reconstitution procedure adopted by the end user. Thus, upon reconstitution of the precursor vial with a suitable aqueous medium, the sucrose dissolves releasing the phospholipid-stabilised PFB microbubbles [Sontum, Ultraso.Med.Biol., 34(5), 824-833 (2008)]. The use of sugars as lyoprotectants or cryoprotectants is well known in the art, and is described in e.g. Solis et al [Int.J.Pharmaceut., 396, 30-38 (2010)] and references therein.

In the method of the first aspect, the size adjustment of step (ii) is preferably carried out by:

(a) one or more cycles of flotation and re-suspension; or
(b) differential centrifugation; or
(c) combinations thereof.

In the method of the first aspect, after step (iii), it is preferred to check that the size distribution and microbubble concentration is suitable, before proceeding to the dispending step (iv) and subsequent lyophilisation. Thus, pass/fail criteria are important at that stage to avoid the time and expense of dispensing and lyophilising unsuitable materials.

In step (iv) of the method of the first aspect, multiple vials are dispensed in parallel - i.e. concurrently. In step (v), multiple filled vials are cooled in parallel - i.e. concurrently. The ordinary person skilled in the art can choose suitable numbers here, based on the dispensing apparatus available (in particular the number of dispenser arms available for concurrent dispensing), the size of the freeze-drier apparatus and the dispensing time per vial. Thus, knowing the dispensing time per vial and the maximum holding time as per the present claims, the skilled person can readily work out how many vials can be filled before the cooling of step (v) must be carried out on a subset of vials within the batch. Preferably, each group of filled vials is progressively loaded into a freeze-drier apparatus (e.g. a shelf within the freeze-drier) maintained at the desired temperature (e.g. -60 °C), and maintained at that temperature until the entire batch has been loaded into the freeze-drier apparatus. In a preferred embodiment 4 filling needles are used from the dispenser, so that 4 vials are filled concurrently. Sub-groups of 70 vials are filled in this manner, then loaded into the freeze-drier pre-cooled to - 60 °C.

A preferred ultrasound contrast agent and precursor of the invention is Sonazoid™ (GE Healthcare AS), previously known as NC100100, as described by Sontum [Ultraso.Med.Biol., 34(5), 824-833 (2008)].

Suitable vials and closures for use in the method of the first aspect are pharmaceutical grade, and suitable for lyophilisation, and are widely available commercially. It is preferred to use a flat-bottomed vial, since that increases the heat transfer between the vial and the shelf of the freeze-drier. The closures are preferably chosen to be suitable for lyophilisation, and designed to permit escape of the vapour out of the vials - making it possible to stopper the vials before the freeze-drier is opened and unloaded.

Sucrose is also commercially available - pharmaceutical grade should be used. Perfluorobutane of pharmaceutical GMP grade can be obtained from F2 Chemicals Limited. H-EPS is commercially available from NOF Corporation, Amagasaki-Shi, Hyogo, Japan.

In a second aspect, the present invention provides a method of preparation of a kit for the preparation of an ultrasound contrast agent which comprises a suspension of microbubbles of perfluorobutane stabilised by hydrogenated egg phosphatidylserine in a sterile, aqueous medium which comprises:

(i) carrying out the method of the first aspect, to give a commercial scale batch of 500 to 80,000 vials containing the contrast agent precursor as defined in the first aspect; and
(ii) provision of a container of sterile, aqueous solution suitable for reconstituting said vial of precursor from step (i) to give said contrast agent.

Preferred embodiments of the method of the first aspect, and precursor in the kit preparation method of the second aspect, are as described in the first aspect (above).

The term "kit" here has its conventional meaning in the field of in vivo imaging, and refers to the imaging agent itself, or a precursor thereto, supplied with all the necessary components to prepare the contrast agent of interest in a form suitable for mammalian administration (as defined above). Such kits are designed to have a usable shelf-life of several weeks or months, such that the clinician can prepare the imaging agent at a suitable juncture and time for imaging the mammalian subject of interest. This is typically more convenient for the clinician, since once prepared, the contrast agent itself will have a much shorter usable shelf-life. Having a supply of the kits means that the clinician has 24-hour, 7-day access to the contrast agent, whenever it is needed. The kit would suitably include a "package leaflet" defining the kit and its components, providing details of how to use the kit, together with patient safety information, and details of the commercial supplier. Such kits typically represent the preferred format of a commercial product designed to provide the ultrasound contrast agent.

The sterile, aqueous solution of step (ii) of the second aspect is preferably a "biocompatible carrier" as defined above. More preferably, it is sterile water for injection. Most preferably, the sterile, aqueous solution has a total concentration of free (i.e. not chelated) aluminium, barium, magnesium, calcium and zinc ions of less than 100 $\mu$M, ideally less than 50 $\mu$M.

The kit of the second aspect preferably further comprises a vented filter (5 $\mu$m) spike such as a Chemoprotect® spike (Codan GmbH & Co, Germany). The kit is reconstituted with the aqueous solution through the spike, followed by manual mixing for about one minute giving a milky, homogeneous dispersion. The dose of contrast agent for imaging the subject is then drawn into a syringe through the filter spike. The role of the "spike" is to remove any extraneous particles or agglomerates - which would be otherwise difficult to detect by visual inspection, since the dispersion within the vial is opaque.

In a third aspect, the present invention provides a method of preparation of an ultrasound contrast agent which comprises a suspension of microbubbles of perfluorobutane stabilised by hydrogenated egg phosphatidylserine in a sterile, aqueous medium which comprises:

> (i) carrying out the method of the first aspect, to give a commercial scale batch of 500 to 80,000 vials containing the contrast agent precursor as defined in the first aspect; and
> (ii) reconstituting the vial of precursor from step (i) with a sterile aqueous solution.

Preferred embodiments of the method of the first aspect, and precursor in the contrast agent preparation method of the second aspect, are as described in the first aspect (above). The method of the third aspect preferably comprises the method of preparation of the kit of the second aspect.

Description of the Figures.

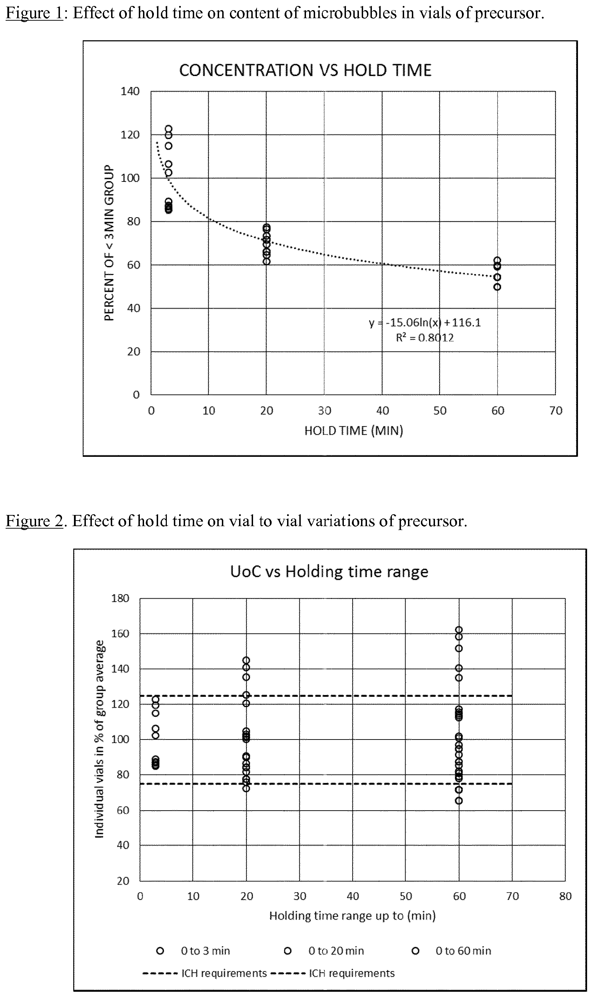

> Figure 1 shows the effect of hold time on the content of microbubbles in vials of precursor - expressed as a percentage of the average level of microbubbles present in vials at a holding time of less than three minutes. The yield of microbubbles per vial was reduced to about 85% of the initial value with a hold time of about 20-minutes, and to only about 75% of the initial value with a hold time of 60 minutes.

> Figure 2 shows the effect of hold time on the UoC of the precursor vials; individual vial values in percent of group average. The longer the hold time range, the greater the diversity of microbubbles per vial. Thus, expressed as relative standard deviation (RSD) the span in content of microbubbles increased from 15% at hold times less than 3 minutes, through to 22% at less than 20 minutes, to as high as 29% at less than 60 minutes. The dashed horizontal lines shows Pharmacopeia requirements to UoC as maximum allowable deviation of individual units from batch average; $\pm$ 25%. As noted, with a hold time range of less than 3 minutes, all vials are within these requirements. Even at a hold time range of 20 minutes, 5 out of 20 vials falls outside of the requirements set in the Pharmacopeia. With a hold time range of 60 minutes 11 out of 39 vials tested are outside requirements.
> In combination, Figures 1 and 2 demonstrate the importance of a short holding time and a narrow holding time variance between filling and lyophilisation, in order to optimise yield and in order to manufacture a product that complies with regulatory requirements to UoC (vial to vial variance).

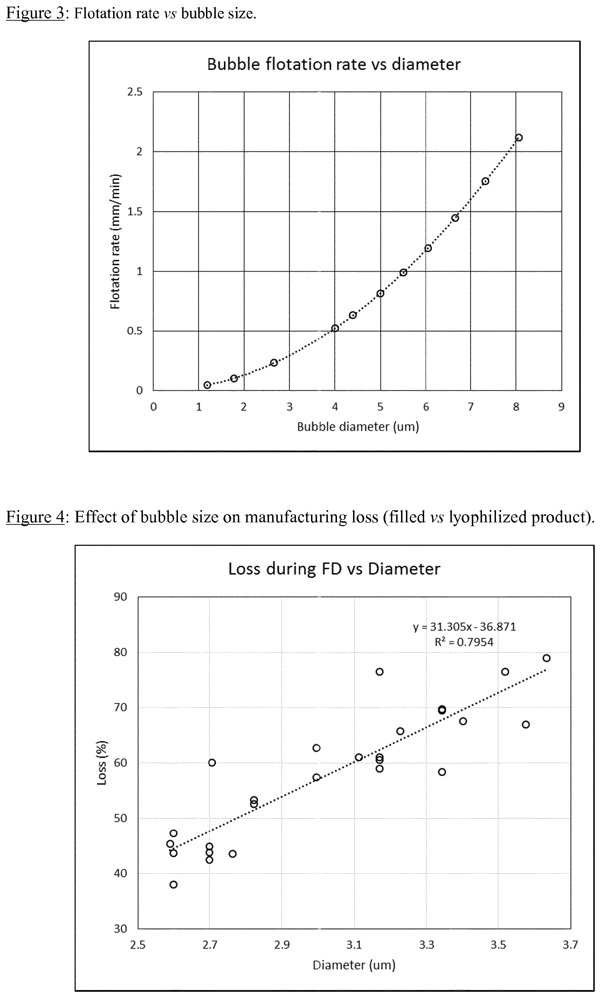

Figure 3 shows the effect of microbubble size (diameter in $\mu$m) on flotation rate. The plot is calculated based on the Stokes equation for a density increment of 1 g/ml and a viscosity of 1 mPa s. Notably, the filling height during lyophilisation is typically 1 cm and all 5 $\mu$m microbubbles in the suspension will hence have segregated to the top of the liquid matrix in approximately 12 minutes.

Figure 4 shows data on the percentage loss of microbubbles vs the size of the microbubbles prior to lyophilisation. Results were from analysis of 10 vials, before and after lyophilisation, for each of 27 batches. Figures 3 and 4 together demonstrate that larger microbubbles cream much more rapidly, and hence lead to loss of yield of microbubbles. Consequently, the UoC in the vials of precursor is more sensitive to the presence of large microbubbles pre-lyophilisation than to smaller ones. Figure 5 also shows that the loss increases from approximately 40% with 2.5 $\mu$m bubbles to 80% with 3.7 $\mu$m bubbles. Figures 3 and 4 together demonstrate why the size adjustment pre-lyophilisation of step (ii) of the method of the first aspect is important.

The invention is illustrated by the non-limiting Examples detailed below. Example 1 provides the preparation of PFB microbubbles stabilised by H-EPS using rotor stator mixing. Example 2 provides a method of size adjustment of the microbubbles of Example 1 using flotation. Example 3 provides details on the effect of holding time on yield and UoC of PFB microbubbles. The results for microbubble concentration vs holding time are shown in Figure 1, and the results for UoC vs holding time range in Figure 2. In combination, Figures 1 and 2 demonstrate the importance of a short holding time and a narrow holding time variance between filling and lyophilisation, in order to optimise yield and in order to manufacture a product that complies with the regulatory requirements as to UoC (vial to vial variance). Example 4 shows the effect of microbubble size on loss during lyophilization. The results for microbubble flotation rate vs diameter are shown in Figure 3 and the results for loss during lyophilisation vs microbubble diameter in Figure 4. In combination, Figures 3 and 4 demonstrate the impact of microbubble size on flotation rate and ensuing increased loss during lyophilisation with increasing microbubble size and hence, the importance of a size adjustment step prior to lyophilisation to minimize loss and to meet the requirements for UoC.

Abbreviations.

FD:        freeze-drying.
GMP:       Good Manufacturing Practice;
H-EPS:     hydrogenated egg phosphatidylserine;

ICH: International Conference on Harmonisation;
i.v.: intravenous;
Min: minutes;
PA: phosphatidic acid;
PFB: perfluorobutane;
PS: phosphatidylserine;
Rpm: revolutions per minute;
UoC: uniformity of content;
WFI: water for injection.

## Example 1: Preparation of Perfluorobutane Microbubble Dispersions by Rotor Stator Mixing.

H-EPS (500.4 mg) was added to 5 to 100 mL water containing 5.4% (w/w) of a mixture of propylene glycol and glycerol (3:10 w/w). The mixture was shaken and heated to 80°C for 5 min, allowed to cool to room temperature, shaken again and left standing overnight prior to use.

A portion (50 mL) of the resulting solution was transferred to a round-bottomed flask with a conical neck. The flask was fitted with a glass jacket having a temperature control inlet and outlet connected to a water bath maintained at 25°C. A rotor stator mixing shaft was introduced into the solution and to avoid gas leakage the space between the neck wall and the mixing shaft was sealed with a specially designed metal plug fitted with a gas inlet/outlet connection for adjustment of gas content and pressure control. The gas outlet was connected to a vacuum pump and the solution was degassed for one minute. An atmosphere of perfluoro-n-butane gas was then applied through the gas inlet. The solution was homogenised at 23,000 rpm for 10 min, keeping the rotor stator mixing shaft such that the openings were slightly above the surface of the liquid. A white-coloured creamy dispersion was obtained, which was transferred to a sealable container and flushed with perfluoro-n-butane. The dispersion was then transferred to a separating funnel and centrifuged at 12,000 rpm for 30 min, yielding a creamy layer of microbubbles at the top and a turbid infranatant. The infranatant was removed and replaced with water. The centrifugation was then repeated twice, but now at 12,000 rpm for 15 min. After the last centrifugation, the supernatant was replaced by 10 % (w/w) sucrose.

## Example 2: Size Adjustment of Perfluorobutane Microbubbles by Flotation.

A cylindrical flat-bottomed glass separation chamber with a total height of 80 cm and an internal diameter of 82 cm was equipped with mechanical stirring blades. The chamber was filled to a height of 6 cm with the microbubble suspension prepared according to Example 1 (32 litres). The suspension was stirred slowly for 2 min, in alternate directions to obtain complete mixing, with speeds varying from 0 to 15 rpm. The suspension was allowed to settle for 200 min, and the bottom 5 cm layer was drained off through 3 outlets in the bottom into a holding tank. A connected laser size-determination apparatus revealed when a particular particle size appeared, and the outlets were closed. Water for injection (WFI) was filled into the separation chamber to 6 cm height, and stirring, settling and drainage cycles were repeated.

Aliquots of the retained particle suspensions after each cycle were tested for size distribution by Coulter Counter measurements. After 11 cycles, where particles above 2 $\mu$m had been retained, further cycles were performed to remove large particles greater than 4 $\mu$m in size, by collecting the lower layer into a receiving tank, and disposing of the upper layer. The settling time was 120 min for these cycles at this stage. After each cycle the contents of the receiving tank were returned to the separation chamber, and WFI was added to 6 cm height.

After a total of 11 cycles to remove under-sized particles, and a subsequent 8 cycles to remove oversized particles, Coulter Counter measurement revealed that 89% of the particles had a size between 2 and 4 $\mu$m.

Using the same apparatus and materials, but with five 200 min separation cycles to remove particles below a desired size limit, then eight 150 min separation cycles to remove particles below a desired size limit and then eight 100 min separation cycles to remove particles above a desired size limit, a narrow fraction was obtained with 92% by number of particles between 2.5 and 4.5 $\mu$m and 72% between 3.0 and 4.0 $\mu$m.

## Example 3: Effect of Holding Time on Yield and UoC - Manufacture of Lyophilized and Size-adjusted PFB Microbubbles.

Portions of 500 ml H-EPSNa dispersion were prepared by adding a solution of 1.5 % (w/w) propylene glycol and 5.1 % (w/w) glycerol 85 % in WFI to 2.5 g H-EPSNa, followed by shaking during heating in a 80°C water bath for 5 min. The mixture was left without stirring at room temperature overnight. The volumes were adjusted to a final concentration of 5 mg/ml H-EPSNa. The phospholipid suspension was autoclaved (F015) and stored at 4°C.

Stabilized PFB microbubbles were prepared by pumping the H-EPSNa suspension together with PFB gas through a continuous sonication process system under the following conditions: PFB-flow 45 ml/min, flow of H-EPSNa dispersion 15 ml/min, frequency 20.000 Hz, and temperature 33°C. The resulting dispersion of stabilized PFB microbubbles was collected in a tank designed for the size adjusting step described below.

WFI (1 kg) was added to the dispersion (2.2 kg). The dispersion was shaken and the microbubbles were allowed to float for 3 h 20 min before 1830 g infranatant (waste) was carefully removed from the bottom, and replaced by 1830 g WFI. The dispersion was shaken and left for another period of 3 h 20 min before removal of 1830 g waste. This procedure was repeated until a total of 8 x 1830 g waste had been removed. The microbubble size-optimized dispersion was adjusted to a final concentration of 3.04 % (v/v) microbubbles and 100 mg/ml sucrose by adding 20% sucrose and WFI. The dispersion was continuously agitated at 200 rpm throughout the filling, and 2 g (1.9-2. 1 g) were filled into 10 ml vials using a pump speed of 100 rpm. The fill weight was recorded every 15-20 min. Trays were loaded with vials (130-140 vials per tray), transported to the freeze-dryer and placed onto pre-cooled shelves (actual temperature -58 ° C).

For each vial, the time between filling and loading into the freeze-dryer (holding time) was recorded. Sample groups were held for < 3 min, 20 min and 60 min prior to loading onto precooled shelves (-58 °C) in the freeze dryer. 10 vials were sampled for each group. After finishing the freeze drying, PFB was added to the vial headspace before stoppering.

The microbubble size distribution and volume concentration was measured by Coulter counting.

## Example 4: Effect of Microbubble Size on Loss During Lyophilisation (Yield)

Manufacture of 27 batches of lyophilized and size-adjusted PFB microbubbles was performed using rotor stator mixing and adjustment of size through the flotation principle (as detailed in Examples 1 and 2 only with slightly different equipment and production settings) prior to lyophilisation. For all these batches, the holding time prior to loading the filled vials into the lyophilizer was up to 20 min.

Samples for Analysis.

For each of the 27 batches, 10 vials before lyophilisation and 10 vials after lyophilisation, were analysed for microbubble content and size distribution by Coulter counting. Hence, the loss in microbubble concentration during lyophilisation, relative to content before lyophilisation was calculated.

Calculation of Flotation rate.

The flotation rate in water of microbubbles vs diameter was calculated based on the Stokes equation:

$$v = 6 * 10^4 * (2 * \rho * g * r^2)/(9 * \eta)$$

Where v is flotation rate in mm/min, $\rho$ is the density increment (set to 1000 kg/m$^3$), g is the gravitational constant (9.8 m/s$^2$), r is the bubble radius in meters and $\eta$ is the viscosity (set to1 mPa s).

## Claims

1. A method of preparation of a commercial scale batch of vials of an ultrasound contrast agent precursor which comprises a lyophilised composition of microbubbles of perfluorobutane stabilised by hydrogenated egg phosphatidylserine in sucrose wherein said batch size is in the range 500 to 80,000 vials; wherein said process comprises:

    (i) provision of an aqueous suspension of said perfluorobutane microbubbles stabilised by hydrogenated egg phosphatidylserine in an aqueous solution;
    (ii) adjusting the size of said microbubbles to a median size in the range 2 to 5 $\mu$m;
    (iii) diluting the size-adjusted suspension from step (ii) with a sterile sucrose aqueous solution to give a final sucrose concentration of 5-20 % w/v;
    (iv) dispensing an aliquot of the composition from step (iii) into a vial to give a filled vial, wherein multiple vials are dispensed in parallel;
    (v) within a holding time period of 1.0 to 2.75 minutes for each filled vial after the dispensing of step (iv) is complete, cooling each such filled vial to a temperature of -40 °C to -70 °C, wherein multiple filled vials are cooled in parallel wherein cooling is achieved by loading into a freeze-drier apparatus, in which the shelves are pre-cooled to -60 °C;
    (vi) repeating steps (iv) and (v) as necessary, until the desired number of cooled, filled vials has been obtained;
    (vii) freeze-drying the cooled, filled vials from steps (v) and (vi);
    (viii) back-filling the headspace gas in the freeze-dried vials from step (vii) with perfluorobutane;
    (ix) sealing the vials from step (viii) with a closure.

2. The method of claim 1, where the lyophilized precursor composition is sterile.

3. The method of claim 1 or claim 2. where the sucrose concentration of step (iii) is 8-12% w/v.

4. The method of any one of claims 1 to 3, where the size adjustment of step (ii) is carried out by:

    (a) one or more cycles of flotation and re-suspension; or
    (b) differential centrifugation; or
    (c) combinations thereof.

5. The method of any one of claims 1 to 4, where after step (iii), the size distribution and microbubble concentration is checked before proceeding to the dispending step (iv).

6. The method of any one of claims 1 to 5, where the holding time period of step (v) is the same for each filled vial.

7. A method of preparation of a kit for the preparation of an ultrasound contrast agent which comprises a suspension of microbubbles of perfluorobutane stabilised by hydrogenated egg phosphatidylserine in a sterile, aqueous medium which comprises:

(i) carrying out the method of any one of claims 1 to 6, to give a commercial scale batch of 500 to 80,000 vials containing the contrast agent precursor as defined in any one of claims 1 to 3; and
(ii) provision of a container of sterile, aqueous solution for each vial suitable for reconstituting said vials of precursor from step (i) to give said contrast agent.

8. The method of claim 7, where the sterile aqueous solution is water for injection which contains less than 100 μM total concentration of free aluminium, barium, magnesium, calcium and zinc ions.

9. A method of preparation of an ultrasound contrast agent which comprises a suspension of microbubbles of perfluorobutane stabilised by hydrogenated egg phosphatidylserine in a sterile, aqueous medium which comprises:

(i) carrying out the method of any one of claims 1 to 6, to give a commercial scale batch of 500 to 80,000 vials containing the contrast agent precursor as defined in any one of claims 1 to 3; and
(ii) reconstituting the vials of precursor from step (i) with a sterile aqueous solution.

**Patentansprüche**

1. Verfahren zur Herstellung einer Charge im kommerziellen Maßstab von Fläschchen eines Ultraschallkontrastmittelvorläufers, der eine lyophilisierte Zusammensetzung von Perfluorbutan-Mikrobläschen umfasst, die durch hydriertes Eiphosphatidylserin in Saccharose stabilisiert sind, wobei die Chargengröße im Bereich von 500 bis 80.000 Fläschchen liegt; wobei der Prozess Folgendes umfasst:

(i) Bereitstellen einer wässrigen Suspension der Perfluorbutan-Mikrobläschen, die durch hydriertes Eiphosphatidylserin in einer wässrigen Lösung stabilisiert sind;
(ii) Anpassen der Größe der Mikrobläschen an eine mittlere Größe im Bereich von 2 bis 5 μm;

(iii) Verdünnen der größenangepassten Suspension von Schritt (ii) mit einer sterilen wässrigen Saccharoselösung, um eine endgültige Saccharosekonzentration von 5-20 % w/v zu erhalten;
(iv) Abfüllen eines Aliquots der Zusammensetzung von Schritt (iii) in ein Fläschchen, um ein gefülltes Fläschchen zu erhalten, wobei mehrere Fläschchen parallel abgefüllt werden;
(v) innerhalb einer Haltezeitdauer von 1,0 bis 2,75 Minuten für jedes gefüllte Fläschchen nach Abschluss des Abfüllens von Schritt (iv) Abkühlen jedes solchen gefüllten Fläschchens auf eine Temperatur von -40 °C bis -70 °C, wobei mehrere gefüllte Fläschchen parallel abgekühlt werden, wobei das Abkühlen durch Laden in eine Gefriertrocknervorrichtung erreicht wird, in der die Regale auf -60 °C vorgekühlt sind;
(vi) Wiederholen von Schritt (iv) und (v) erforderlichenfalls, bis die gewünschte Anzahl gekühlter, gefüllter Fläschchen erhalten wurde;
(vii) Gefriertrocknen der gekühlten, gefüllten Fläschchen von Schritt (v) und (vi);
(viii) Auffüllen des Kopfraumgases in den gefriergetrockneten Fläschchen von Schritt (vii) mit Perfluorbutan;
(ix) Verschließen der Fläschchen von Schritt (viii) mit einem Verschluss.

2. Verfahren nach Anspruch 1, wobei die lyophilisierte Vorläuferzusammensetzung steril ist.

3. Verfahren nach Anspruch 1 oder Anspruch 2, wobei die Saccharosekonzentration von Schritt (iii) 8-12 % w/v beträgt.

4. Verfahren nach einem der Ansprüche 1 bis 3, wobei die Größenanpassung von Schritt (ii) durch Folgendes durchgeführt wird:

(a) einen oder mehrere Flotations- und Resuspensionszyklen; oder
(b) Differentialzentrifugation oder
(c) Kombinationen davon.

5. Verfahren nach einem der Ansprüche 1 bis 4, wobei nach Schritt (iii) die Größenverteilung und die Mikrobläschenkonzentration überprüft werden, bevor mit dem Abfüllschritt (iv) fortgefahren wird.

6. Verfahren nach einem der Ansprüche 1 bis 5, wobei die Haltezeitdauer von Schritt (v) für jedes gefüllte Fläschchen gleich ist.

7. Verfahren zur Herstellung eines Kits für die Herstellung eines Ultraschallkontrastmittels, das eine Suspension von Perfluorbutan-Mikrobläschen umfasst, die durch hydriertes Eiphosphatidylserin in einem sterilen, wässrigen Medium stabilisiert sind, das Folgendes umfasst:

(i) Durchführen des Verfahrens nach einem der Ansprüche 1 bis 6, um eine Charge im kommerziellen Maßstab von 500 bis 80.000 Fläschchen zu erhalten, die den Kontrastmittelvorläufer nach einem der Ansprüche 1 bis 3 enthalten; und

(ii) Bereitstellen eines Behälters mit steriler, wässriger Lösung für jedes Fläschchen, der zur Rekonstitution der Vorläuferfläschchen von Schritt (i) geeignet ist, um das Kontrastmittel zu erhalten.

8. Verfahren nach Anspruch 7, wobei es sich bei der sterilen wässrigen Lösung um Wasser zur Injektion handelt, das weniger als 100 $\mu$m Gesamtkonzentration an freien Aluminium-, Barium-, Magnesium-, Calcium- und Zinkionen enthält.

9. Verfahren zur Herstellung eines Ultraschallkontrastmittels, das eine Suspension von Perfluorbutan-Mikrobläschen umfasst, die durch hydriertes Eiphosphatidylserin in einem sterilen, wässrigen Medium stabilisiert sind, das Folgendes umfasst:

(i) Durchführen des Verfahrens nach einem der Ansprüche 1 bis 6, um eine Charge im kommerziellen Maßstab von 500 bis 80.000 Fläschchen zu erhalten, die den Kontrastmittelvorläufer nach einem der Ansprüche 1 bis 3 enthalten; und

(ii) Rekonstituieren der Vorläuferfläschchen von Schritt (i) mit einer sterilen wässrigen Lösung.

## Revendications

1. Procédé de préparation d'un lot à échelle commerciale de flacons d'un précurseur d'agent de contraste ultrasonore qui comprend une composition lyophilisée de microbulles de perfluorobutane stabilisées par de la phosphatidylsérine d'œuf hydrogénée dans du saccharose, ladite taille de lot étant comprise entre 500 et 80 000 flacons ; ledit procédé comprenant :

(i) la fourniture d'une suspension aqueuse desdites microbulles de perfluorobutane stabilisées par de la phosphatidylsérine d'œuf hydrogénée dans une solution aqueuse ;
(ii) l'ajustement de la taille desdites microbulles à une taille médiane comprise entre 2 et 5 $\mu$m ;
(iii) la dilution de la suspension ajustée de taille de l'étape (ii) avec une solution aqueuse de saccharose stérile pour obtenir une concentration finale en saccharose de 5-20 % p/v ;

(iv) la distribution d'une aliquote de la composition de l'étape (iii) dans un flacon pour donner un flacon rempli, de multiples flacons étant distribués en parallèle ;
(v) dans un temps de maintien de 1,0 à 2,75 minutes pour chaque flacon rempli après que la distribution de l'étape (iv) est terminée, le refroidissement de chaque flacon rempli à une température de -40 °C à -70 °C, de multiples flacons remplis étant refroidis en parallèle, le refroidissement étant obtenu par chargement dans un appareil lyophilisateur dans lequel les étagères sont prérefroidies à -60 °C ;
(vi) la répétition des étapes (iv) et (v) si nécessaire, jusqu'à obtention du nombre souhaité de flacons remplis refroidis ;
(vii) la lyophilisation des flacons remplis refroidis des étapes (v) et (vi) ;
(viii) le remplissage par du gaz de l'espace de tête dans les flacons lyophilisés de l'étape (vii) avec du perfluorobutane ;
(ix) le scellement des flacons de l'étape (viii) avec un bouchon.

2. Procédé selon la revendication 1, la composition précurseur lyophilisée étant stérile.

3. Procédé selon la revendication 1 ou la revendication 2, la concentration en saccharose de l'étape (iii) étant 8-12 % p/v.

4. Procédé selon l'une quelconque des revendications 1 à 3, l'ajustement de taille de l'étape (ii) étant effectué par :

(a) un ou plusieurs cycles de flottation et de remise en suspension ; ou
(b) la centrifugation différentielle ; ou
(c) des combinaisons de ceux-ci.

5. Procédé selon l'une quelconque des revendications 1 à 4, après l'étape (iii), la distribution de taille et la concentration de microbulles étant vérifiées avant de passer à l'étape de distribution (iv).

6. Procédé selon l'une quelconque des revendications 1 à 5, la durée de conservation de l'étape (v) étant la même pour chaque flacon rempli.

7. Procédé de préparation d'un kit pour la préparation d'un agent de contraste ultrasonore qui comprend une suspension de microbulles de perfluorobutane stabilisées par de la phosphatidylsérine d'œuf hydrogénée dans un milieu aqueux stérile qui comprend :

(i) la mise en œuvre du procédé selon l'une quelconque des revendications 1 à 6, pour donner un lot à échelle commerciale de 500 à 80 000 flacons contenant le précurseur d'agent de contraste tel que défini selon l'une quelconque des revendications 1 à 3 ; et
(ii) la fourniture d'un récipient de solution aqueuse stérile pour chaque flacon approprié pour reconstituer lesdits flacons de précurseur de l'étape (i) pour donner ledit agent de contraste.

8. Procédé selon la revendication 7, la solution aqueuse stérile étant de l'eau pour injection qui contient moins de 100 pM de concentration totale d'ions libres d'aluminium, baryum, magnésium, calcium et zinc.

9. Procédé de préparation d'un agent de contraste ultrasonore qui comprend une suspension de microbulles de perfluorobutane stabilisées par de la phosphatidylsérine d'œuf hydrogénée dans un milieu aqueux stérile qui comprend :

(i) la mise en œuvre du procédé selon l'une quelconque des revendications 1 à 6, pour donner un lot à échelle commerciale de 500 à 80 000 flacons contenant le précurseur d'agent de contraste tel que défini selon l'une quelconque des revendications 1 à 3 ; et
(ii) la reconstitution des flacons de précurseur de l'étape (i) avec une solution aqueuse stérile.

Figure 1: Effect of hold time on content of microbubbles in vials of precursor.

Figure 2. Effect of hold time on vial to vial variations of precursor.

Figure 3: Flotation rate *vs* bubble size.

Figure 4: Effect of bubble size on manufacturing loss (filled *vs* lyophilized product).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9729782 A
- WO 9903558 A
- WO 9908715 A

- WO 9908716 A
- EP 1228770 A1
- WO 2004069284 A

### Non-patent literature cited in the description

- **WHEATLEY et al.** *J.Drug Del.Sci.Technol*, 2013, vol. 23 (1), 57-72
- **SOLIS et al.** *Int.J.Pharmaceut*, 2010, vol. 396 (1-2), 30-38
- **FESHITAN et al.** *J. Coll.Interf. Sci.*, 2009, vol. 329, 316-324
- **SIRSI et al.** *, Bubble Sci.Eng.Technol*, 2009, vol. 1 (1-2), 3-17
- **HVATTUM et al.** *J.Pharm.Biomed. Anal*, 2006, vol. 42, 506-512
- **STRIDE et al.** *Soft Matter*, 2008, vol. 4, 2350-2359
- **UNNIKRISHNAN et al.** *Am.J.Roentgenol.*, 2012, vol. 199 (2), 292-299
- **WANG et al.** *Ultraso.Med.Biol*, 2013, vol. 39 (5), 882-892

- **RODGERS et al.** *Chem.Eng.Res.Rev*, 2012, vol. 90 (3), 323-327
- Emulsion Formation and Stability. Wiley VCH, 2013
- **PACEK et al.** Emulsification in Rotor-Stator Mixers, 127-167
- **KVALE et al.** *Separat.Technol.*, 1996, vol. 6, 219-226
- **FESHITAN et al.** *J.Coll.Interf.Sci*, 2009, vol. 329, 316-324
- **SONTUM.** *Ultraso.Med.Biol*, 2008, vol. 34 (5), 824-833
- **SOLIS et al.** *Int.J.Pharmaceut.*, 2010, vol. 396, 30-38